# EUROPEAN PATENT SPECIFICATION

(11) **EP 1 080 158 B1**
(45) Date of publication and mention of the grant of the patent: **11.09.2002**
(21) Application number: 99915887.6
(22) Date of filing: 08.04.1999
(51) Int. Cl.: C09J 189/00

(54) **ADHESIVES**
KLEBSTOFFE
ADHESIFS

(30) Priority: 09.04.1998 GB 9807777
(43) Date of publication of application: 07.03.2001
(73) Proprietor: MARS UK LIMITED, Slough, Berkshire SL1 4LW (GB); BTTG, Leeds LS16 6QL (GB)
(72) Inventor: NELSON, Gordon, Sale, Cheshire M33 2FJ (GB); JONES, Christopher, Andrew, West Bridgford, Nottingham NG2 5AB (GB)
(74) Representative: Davies, Jonathan Mark
(86) International application number: GB9901080
(87) International publication number: WO99052988

(56) References cited:
- EP-A- 0 244 688
- WO-A-96/20284
- US-A- 5 202 256
- PATENT ABSTRACTS OF JAPAN vol. 18, no. 316 (C-1213), 16 June 1994 (1994-06-16) & JP 06 070972 A (NIPPON B X I KK), 15 March 1994 (1994-03-15) & DATABASE WPI Week 9415 Derwent Publications Ltd., London, GB; AN 123366
- HIROYUKI YAMAMOTO ET AL.: "Cross-linking and gel-formation of water-soluble lysine polypeptides." INTERNATION JOURNAL OF BIOLOGICAL MACROMOLECULES, vol. 14, no. 2, 1 April 1992 (1992-04-01), pages 66-72, XP002108796 London
- PATENT ABSTRACTS OF JAPAN vol. 17, no. 180 (C-1046), 8 April 1993 (1993-04-08) & JP 04 334396 A (HITACHI CHEM CO LTD), 20 November 1992 (1992-11-20) & DATABASE WPI Week 9301 Derwent Publications Ltd., London, GB; AN 5540

## Description

The present invention relates to water resistant adhesives.

Adhesives are widely used both domestically and industrially. They are typically applied to dry first and second surfaces to bond the surfaces together, for example as bonding agents to bond together particulate matter, or to adhere other solid materials such as woods and metals. It is often desired to bond surfaces together when one or more of the surfaces is wet. Many adhesives are, however, less effective or ineffective if the surfaces that are to be bonded together are wet or if water is applied to the bond after it has been formed. Consequently, much effort has been spent trying to identify and develop adhesives which are effective in wet environments.

Marine mussels are able to attach themselves to a variety of surfaces under water forming strong and durable bonds with those surfaces. The precise mechanism by which mussels achieve this adhesion is not known. J.H.Waite has described the proposed involvement of catechol oxidase and a phenolic protein (Mussel Beards: a coming of age. Chemistry & Industry, 1991, 607-611). The mussel polyphenolic protein is a highly repetitive protein which comprises 80 tandem repeats of a decapeptide of the sequence: Ala-Lys-Pro-Ser-Tyr-Pro-Pro-Thr-Tyr-Lys. The mussel protein is particularly rich in the amino acid 3,4-dihydroxyphenyl-L-alanine (L-dopa). Catechol oxidase occurs extensively throughout nature and is known to catalyse the ortho hydroxylation of phenols and oxidation of the resulting catechols to o-quinones. Waite proposed, therefore, that catechol oxidase catalyses oxidation of the L-dopa residues in the mussel polyphenolic protein to L-quinone. It was suggested that adhesion results from a combination of coupling of peptidyl-dopa to the bound surface and crosslinking of the polyphenolic protein by reaction of peptidyl-dopa-quinone with nucleophiles such as the å-amino group of lysine residues in the protein.

A catechol oxidase from mushrooms known as mushroom tyrosinase is available commercially and has been shown to hydroxylate tyrosine residues in synthetic decapeptides identical in sequence to the repeat sequences in the polyphenolic protein (Marumo K. and Waite J.H. (1986) Optimization of hydroxylation of tyrosine and tyrosine-containing peptides by mushroom tyrosinase. *Biochemica et Biophysica* Acta **872,** 98-103).

Large scale production of the mussel polyphenolic protein has been attempted with a view to production of a commercial adhesive which can be crosslinked by mushroom tyrosinase. Methods for isolation of the polyphenolic protein from mussels are described in US 4 496 397, US 4 585 585, and US 4 687 740. None of these methods, however, have proved to be commercially viable because the polyphenolic protein can only be isolated in small quantities.

WO 97/19141 describes a method of manufacture of an adhesive comprising crosslinking first and second molecules such as gelatin and chitosan, each having one or more aromatic groups, via at least one quinone group. Gelatin and chitosan are readily available and adhesives formed by this method show some resistance to water. However, the bonds formed are not as strong and durable as those formed by the mussel polyphenolic protein. A further disadvantage is that gelatin is not soluble in water at room temperature and has to be heated before use. Chitosan is only soluble at low pH.

It is desired therefore to produce bioadhesives which are effective in wet environments, which can be produced cheaply in bulk quantities, and which have improved properties over known readily produced bioadhesives.

Extensin proteins are hydroxyproline-rich glycoproteins present in the cell walls of dicotyledonous plants. The exact function of extensins is not known, but they have been proposed to play a role in the structure of plant cell walls. Extensins also accumulate in plant cell walls upon wounding and pathogenic attack and are therefore also thought to be involved in defence. It is known that extensins are inherently sticky and their adhesion to glass, polypropylene, and polycarbonate has been described in a paper by Miller, J.G. and Fry, S.C., (1993) (Spinach extensin exhibits characteristics of an adhesive polymer. *Acta Bot. Neerl.* **42**(2), 221-231). However, the mechanism of adhesion here appears to be a mixture of purely intramolecular forces such as hydrogen bonding and ionic interactions. Oxidative processes are not involved as the adhesion is not inhibited using reducing agents such as ascorbate, mercaptoethanol and dithiothreitol. Although native non-crosslinked proteins can adhere to inert substances, the set adhesive will be sensitive to moisture. Ultimately strength loss and bond breakage will become apparent. A successful commercial adhesive system must be crosslinked both to improve the strength of the adhesive and to ensure adhesion in wet environments and areas of high moisture.

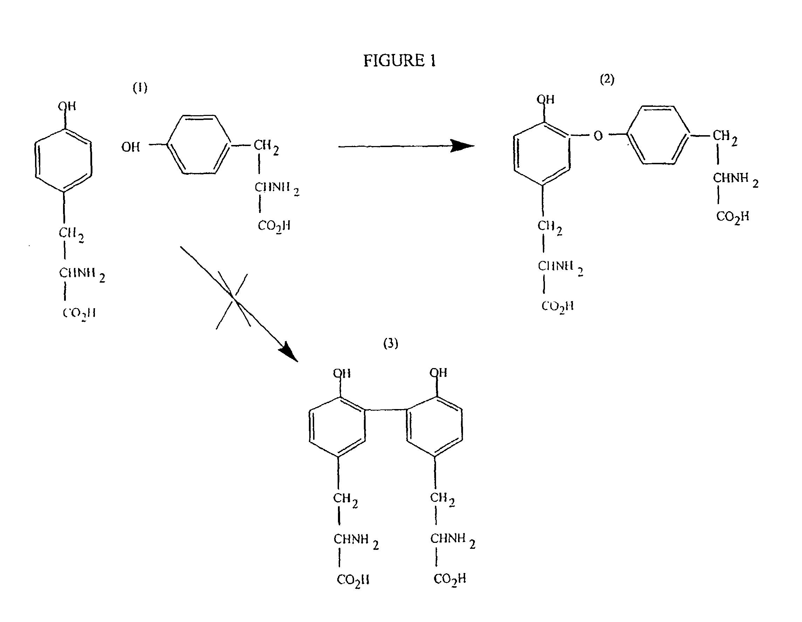

Extensins have been proposed to form crosslinks by Fry, C.F., (1982) (Isodityrosine, a new crosslinking amino acid from plant cell wall glycoprotein. Biochem.J. **204,** 449-455). Fry suggested that hydrogen peroxide and a peroxidase enzyme such as horseradish peroxidase could be used to form isodityrosine bonds via an ether linkage between two tyrosine residues. Formation of dityrosine crosslinks via a biphenyl linkage between two tyrosines was not thought to occur. The formation of an isodityrosine bond and comparison of that linkage with the dityrosine linkage is represented in figure 1 of the accompanying drawings.

The proposed existence of such crosslinking suggests that extensins may form adhesives in the presence of hydrogen peroxide and a peroxidase enzyme. However, it is most unlikely that formation of an adhesive based on addition of a cofactor such as hydrogen peroxide will be practical. Hydrogen peroxide is a relatively low viscosity liquid and would be very difficult to mix with other components of the adhesive which also tend to be relatively viscous. Hydrogen peroxide is also fairly reactive and it is likely that bond formation would occur too quickly after it is added to the other components of the adhesive. A further disadvantage of hydrogen peroxide is that it has a relatively short shelf-life. Any contamination from, for example, dirt would introduce bacteria, many of which contain catalase enzymes which breakdown hydrogen peroxide.

Peroxidase has also been proposed to be involved in the hydroxylation of L-tyrosine residues to L-Dopa (Klibanov A.M., Berman Z., and Alberti B.N. (1981) Preparative hydroxylation of aromatic compounds catalysed by peroxidase. *J.Am.Chem.Soc.* **103,** 6263-6264), suggesting an alternative role for peroxidase in crosslink formation similar to crosslink formation in the mussel polyphenolic protein adhesive. However, this reaction requires oxygen and dihydroxyfumaric acid and must be carried out at 0°C, otherwise non specific oxidation of other amino acids occurs. Use of peroxidase for crosslinking of extensin proteins has therefore not been thought to provide a viable way of producing a commercial adhesive.

It has surprisingly been found that extensin proteins have remarkable adhesive properties.

According to a first aspect of the invention there is provided a composition for use as an adhesive comprising:
an extensin protein; and
a non-enzymatic bifunctional crosslinking agent; and/or
a phenol oxidase and a phenol hydroxylase.

According to the invention there is also provided a method for forming an adhesive according to the first aspect of the invention which comprises admixing an extensin protein with either an amount of:
a non-enzymatic bifunctional crosslinking agent; and/or
a phenol oxidase and a phenol hydroxylase effective for inducing crosslinking of the protein.

Each component of compositions according to the invention may be soluble in water.

When using a phenol oxidase and a phenol hydroxylase for crosslinking, it is possible to include as a cofactor a phenolic moiety which comprises at least one of a monohydroxy phenol group or a dihydroxy phenol group. Examples of phenolic moieties which comprise a dihydroxy phenol group include catechol and catechin. The cofactor should be soluble in water. The cofactor may be present at about 1% weight by volume of the composition.

The term "extensin protein" used herein is defined for the purposes of this application as covering:
(i) natural extensin proteins, such as plant extensins (for example carrot, spinach, etc.);
(ii) non-natural synthetic extensins, such as extensins produced chemically or by expression of recombinant DNA in a suitable host;
(iii) extensin derivatives (whether chemical or synthetic) which have amino acid sequences which differ from the extensin sequences by virtue of amino acid substitution, deletion, or addition, protease truncation or post translational modification; but which retain extensin activity.

Natural extensin protein as referred to in (i) above is a plant protein rich in hydroxyproline, tyrosine and lysine residues. The content of hydroxyproline in carrot extensin is at least 50%, the content of tyrosine is at least 10.1%, and the content of lysine is at least 6.9%.

DNA encoding carrot extensin has been cloned (Chen J. and Varner J.E. (1985) An extracellular matrix protein in plants: characterisation of a genomic clone for carrot extensin. EMBO *J.* **4,** 2145-2151; Chen J. and Varner JE (1985) Isolation and characterisation of cDNA clones for carrot extensin and a proline-rich 33KDa protein. *Proc.Natl.Acad.Sci.USA* **82,** 4399-4403).

Derivatives of the extensin as referred to in (iii) above may be obtained by expression of a modified DNA including a modified extensin gene. A derivative of the extensin may be substantially free of carbohydrate.

The extensin protein may be present in the composition in an amount upto about 50% weight by volume of the composition, for example, the extensin protein may be present in the composition in an amount from about 20% to about 30% weight by volume of the composition.

A procedure for isolation of an extensin according to the invention from carrots is described below:

Carrot extensin proteins were isolated from cores (7mm) of phloem parenchyma sliced with a scalpel into 1mm thick slices. Carrot preparations (90g) (finely chopped or gently homogenised) were washed twice in distilled water and incubated in potassium phosphate buffer (5mM, pH 6.0) containing chloramphenicol (50mg/ml) for three days at 28°C with shaking. The extraction buffer was exchanged each day. On completion, the carrots were washed in distilled water and immersed in a solution (600ml) containing polyvinyl polypyrrolidone (PVPP) (9g) and dithiothreitol (DTT) (5mM, final concentration). The tissue was homogenised for two minutes in a blender, pelleted by centrifugation and washed with eight litres of distilled water. The cell wall pellet was further extracted (3 times) with 200ml of a solution containing calcium chloride (0.2M final concentration), DTT (5mM final concentration) and PVPP (9g). On centrifugation the supernatants were pooled, filtered to remove any solid material and reduced to a volume of 50ml using ultrafiltration (10,000 molecular weight cut-off). The concentrated extract was dialysed overnight in distilled water at 4°C and further reduced in volume to 10ml by ultrafiltration. Each 10ml volume was adjusted with 1M tris/HCl (pH 8) to give a final tris concentration of 10mM. The material was then applied to a cation exchange column (12 x 1.5 cm) (CM-Sepharose CL-6B) previously equilibrated with tris/HCl (10mM, pH 8) and the protein fractions eluted using one column volume of tris/HCl (pH 8) followed by a linear gradient (60ml) of 10-300mM tris/HCl (pH 8). The elution profile was monitored at 280nm and the fractions (3ml) corresponding to each peak were pooled and dialysed overnight with sodium acetate (100mM, pH 6) at 4°C. The dialysed samples were freeze-dried and weighed before further analysis. In all purifications peak 1 from the cation exchange column was ignored as it was primarily made up of PVPP.

It is noted herein that peroxidases are not phenol oxidases. Peroxidases act by hydrolysing hydrogen peroxide. It is possible that oxygen liberated by this reaction can oxidise phenolics, but this oxidation does not occur by an enzymatic process. Phenol oxidation is not possible in the presence of a peroxidase without the addition of hydrogen peroxide. It is also noted herein that peroxidases are not phenol hydroxylases.

The phenol oxidase and the phenol hydroxylase may be a tyrosinase. The composition can contain at least 0.005% weight by volume of tyrosinase. Tyrosinase has the advantage that it is readily available from a variety of sources. The tyrosinase may be a mushroom tyrosinase. The mushroom tyrosinase may be *Agaricus bisporus* tyrosinase.

The non-enzymatic bifunctional crosslinking agent may be present in the composition from about 0.1% to about 5% weight by volume of the composition. The non-enzymatic bifunctional crosslinking agent may be any non-enzymatic bifunctional crosslinking agent capable of inducing crosslinking of a composition for use as an adhesive according to the invention. The non-enzymatic bifunctional crosslinking agent may be at least one of glutaraldehyde, a di-isocyanate, or a quinone. The di-isocyanate may be Trixene, for example Trixene BI 7986 (Baxenden). Trixene has the advantage that it is blocked and unreactive until the temperature reaches 130°C. Compositions according to the invention that comprise Trixene may therefore form a thermo-setting adhesive. The quinone may be a benzoquinone or a derivative thereof. The amount of the benzoquinone in the composition can be about 1% weight by volume of the composition. The benzoquinone may be 1,2-benzoquinone, 1,3-benzoquinone, or 1,4-benzoquinone.

It has been found that the rate of oxidation of groups may be conveniantly controlled by varying the amount of phenol oxidase and phenol hydroxylase that is added, allowing the rate of linkage to be readily controlled. The rate of linkage may also be controlled by varying the amount of the non-enzymatic bifunctional crosslinking agent.

When compositions according to the invention that contain the non-enzymatic bifunctional crosslinking agent form crosslinks, the non-enzymatic bifunctional crosslinking agent reacts with the ε-amino group of lysine residues in the extensin protein. Each molecule of non-enzymatic bifunctional crosslinking agent can react with upto two lysine residues. When the two lysine residues are in different protein molecules, crosslinks are formed between different protein molecules. When the two lysine residues are in the same protein molecule, crosslinks are formed within the same protein molecule.

When compositions according to the invention that comprise the phenol oxidase and the phenol hydroxylase form crosslinks, the phenol hydroxylase catalyses the hydroxylation of the phenol group of tyrosine residues in the protein to form a dihydroxy phenol group and the phenol oxidase catalyses the oxidation of the dihydroxy phenol group to form a quinone group. The quinone group of the modified tyrosine residue may then react with the ε-amino group of two lysine residues in the protein. When one or both of the lysine residues are in the same protein molecule as the modified tyrosine residue containing the quinone group, intramolecular crosslinking occurs. When one or both of the lysine residues are in different protein molecules to the modified tyrosine residue containing the quinone group, intermolecular crosslinking occurs.

Tyrosinase has the advantage that it is a phenol hydroxylase and a phenol oxidase. It will be appreciated therefore that compositions according to the invention which comprise tyrosinase have the advantage that a separate phenol oxidase and a phenol hydroxylase do not have to be added to the composition because the phenol oxidase and the phenol hydroxylase activity are on a single molecule.
Figure 1 shows the formation of an isodityrosine bond and comparison of that linkage with the dityrosine linkage;
Figure 2 shows the hydroxylation and oxidation of a tyrosine residue by tyrosinase and reaction of the oxidised tyrosine residue with the ε-amino group of a lysine residue in the same protein molecule.

When compositions according to the invention that comprise the phenol oxidase and the phenol hydroxylase further include a cofactor which comprises a phenolic moiety with a monohydroxy phenol group, the phenol hydroxylase catalyses the hydroxylation of the monohydroxy phenol group in the cofactor to form a dihydroxy phenol group and the phenol oxidase catalyses the oxidation of the dihydroxy phenol group to form a quinone group. The quinone group of the modified cofactor may then react with the ε-amino group of lysine residues in the protein to form crosslinks within the same protein molecule or between different protein molecules.

When compositions according to the invention that comprise the phenol oxidase and the phenol hydroxylase further include a cofactor which comprises a phenolic moiety with a dihydroxy phenol group, the phenol oxidase catalyses the oxidation of the dihydroxy phenol group of the cofactor to form a quinone group. The quinone group of the modified cofactor may then react with the ε-amino group of lysine residues in the protein to form crosslinks within the same protein molecule or between different protein molecules. Thus, a cofactor comprising a monohydroxy phenol group or a dihydroxy phenol group may be used to increase the number of crosslinks formed compared to compositions according to the invention which comprise the phenol oxidase and the phenol hydroxylase that do not include the cofactor. The number of crosslinks which can potentially be formed may also be residues in the extensin protein by recombinant means. However, care should be taken not to increase the number of crosslinks formed too much as over-crosslinked adhesives can be brittle.

It will be appreciated that compositions according to the invention that comprise a cofactor which includes a dihydroxy phenol group may form crosslinks in the absence of the phenol hydroxylase. Consequently, there is also provided according to the invention a composition for use as an adhesive which comprises:
an extensin protein;
a cofactor comprising a dihydroxy phenol group;
a phenol oxidase; and optionally
a non-enzymatic bifunctional crosslinking agent.

There is also provided a method for forming an adhesive which comprises admixing an extensin protein with an amount of a cofactor comprising a dihydroxy phenol group, a phenol oxidase, and optionally a non-enzymatic bifunctional crosslinking agent effective for inducing crosslinking of the protein.

According to the invention there is also provided a kit for manufacture of an adhesive, the kit comprising separate components, wherein admixture of the separate components forms a composition according to the invention.

According to the invention there is also provided a kit for manufacture of an adhesive that comprises separate first and second components, the first component comprising an extensin protein, the second component comprising: a non-enzymatic bifunctional crosslinking agent; and/or
a phenol oxidase and a phenol hydroxylase and optionally a cofator;
wherein admixture of the first and second components forms a composition according to the invention.

Adhesives formed using compositions according to the invention have excellent strength, durability and water resistant properties. They are readily produced in bulk quantities and at low cost. The strength of adhesives according to the invention has been found to be at least as good as the strength of conventional water resistant adhesives.

Adhesives according to the invention have been found to have several uses. Adhesives according to the invention may adhere to water-absorbent substrates and can be used to adhere such substrates together. Adhesives of the present invention may adhere to a substrate having a hydroxy aromatic, dihydroxy aromatic, hydroxy phenone or amino group and can be used to adhere such substrates together. For example the substrate may be wood, leather, cotton, paper, carpet, or a textile.

Adhesives according to the invention have also been found to adhere to non water-absorbent substrates and can be used to adhere non water-absorbent substrates to each other or to water absorbent substrates. For example, the non water-absorbent substrate may be a metal or a plastic.

Adhesives according to the invention may be used to bind together particulates. Examples of particulates include sand and glass fibre.

Adhesives according to the invention may be used as an undercoat to a protective coating such as a paint or to a coating such as a non water-resistant adhesive. Undercoats comprising adhesives according to the invention have been found to have the advantage that the coating that is subsequently applied to the undercoat is significantly more resistant to the effects of water than coatings applied in the absence of the undercoat or to coatings applied to a conventional undercoat.

Adhesives according to the invention may be used as a suture to close wounds. Sutures formed according to the invention have the advantage that they are resistant to water and may have reduced antigenicity compared to conventional sutures.

Adhesives according to the invention may be used as a gelling agent in food products.

According to the invention there is also provided a pharmaceutical composition comprising a pharmaceutically active ingredient and a crosslinked adhesive composition according to the invention.

### Figure Legends

Figure 1 shows the formation of an isodityrosine crosslink (in compound (2)) by hydrogen peroxide and peroxidase from two tyrosines (1). Formation of a dityrosine crosslink (in compound (3)) is not thought to occur.

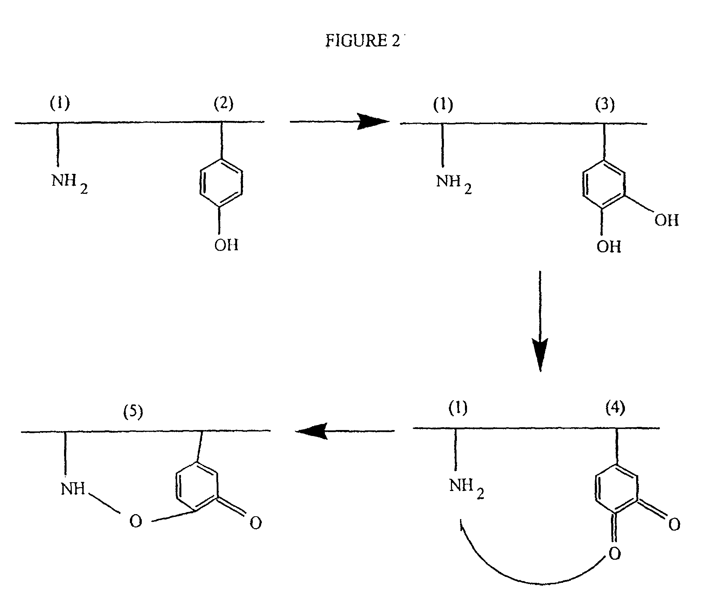

Figure 2 shows a tyrosine residue (2) being hydroxylated (3) and subsequently oxidised(4) by tyrosinase, followed by reaction of the oxidised tyrosine residue (4) with the ε-amino group of a lysine residue (1) in the same protein molecule to form an intramolecular cross link (5).

## Claims

1. A composition for use as an adhesive comprising:
an extensin protein; and
a non-enzymatic bifunctional crosslinking agent; and/or
a phenol oxidase and a phenol hydroxylase.

2. A composition according to claim 1 which further comprises a cofactor when the composition comprises a phenol oxidase and a phenol hydroxylase.

3. A method for forming an adhesive which comprises admixing an extensin protein with either an amount of:
a non-enzymatic bifunctional crosslinking agent; and/or
a phenol oxidase and a phenol hydroxylase effective for inducing crosslinking of the protein.

4. A method according to claim 3 in which the extensin protein is admixed with a cofactor when the adhesive comprises a phenol oxidase and a phenol hydroxylase, the amount of the cofactor, phenol oxidase and phenol hydroxylase being effective for inducing crosslinking of the protein or the amount of the cofactor, non-enzymatic bifunctional crosslinking agent, phenol oxidase and phenol hydroxylase being effective for inducing crosslinking of the protein.

5. A composition or method according to claim 2 or 4 in which the cofactor comprises a phenolic moiety which comprises at least one of a monohydroxy phenol group or a dihydroxy phenol group.

6. A composition or method according to claim 2, 4, or 5 in which the cofactor is soluble in water.

7. A composition or method according to claim 5 or 6 in which the cofactor comprises catechin.

8. A composition or method according to any of claims 5 to 7 in which the cofactor comprises catechol.

9. A composition or method according to any preceding claim in which the non-enzymatic bifunctional crosslinking agent comprises glutaraldehyde.

10. A composition or method according to any preceding claim in which the non-enzymatic bifunctional crosslinking agent comprises a di-isocyanate.

11. A composition or method according to claim 10 in which the di-isocyanate is Trixene.

12. A composition or method according to any preceding claim in which the non-enzymatic bifunctional crosslinking agent comprises a quinone.

13. A composition or method according to claim 12 in which the quinone is a benzoquinone.

14. A composition or method according to any preceding claim in which the phenol oxidase and the phenol hydroxylase is a tyrosinase.

15. A composition or method according to claim 14 in which the tyrosinase is a mushroom tyrosinase.

16. A composition or method according to claim 15 in which the mushroom tyrosinase is *Agaricus bisporus* tyrosinase.

17. A composition for use as an adhesive which comprises:
an extensin protein;
a cofactor comprising a dihydroxy phenol group;
a phenol oxidase; and optionally
a non-enzymatic bifunctional crosslinking agent.

18. A method for forming an adhesive which comprises admixing an extensin protein with an amount of a cofactor comprising a dihydroxy phenol group, a phenol oxidase, and optionally a non-enzymatic bifunctional crosslinking agent effective for inducing crosslinking of the protein.

19. Use of a composition according to any of claims 1, 2, or 5-17 for binding substrates together.

20. Use according to claim 19 in which the substrates are non water-absorbent.

21. Use according to claim 19 in which the substrates are water absorbent.

22. Use according to claim 19 in which the substrates comprise a non water-absorbent substrate and a water absorbent substrate.

23. Use according to claim 20 or 22 in which the non water-absorbent substrate or substrates comprise at least one of metal or plastic.

24. Use according to claim 21 or 22 in which the water absorbent substrate or substrates comprise at least one of wood, leather, cotton, paper, carpet, or textile.

25. Use according to claim 19 as a binder of particulates.

26. Use according to claim 25 in which the particulates comprise at least one of sand or glass fibre.

27. Use according to claim 19 as an undercoat to a coating.

28. Use according to claim 27 in which the coating is a paint.

29. Use according to claim 27 in which the coating is an adhesive.

30. Use according to claim 19 as a suture for closing a wound.

31. Use according to claim 30 in a method for closing a wound.

32. Use according to claim 19 as a gelling agent in food products.

33. A pharmaceutical composition comprising a pharmaceutically active ingredient and a crosslinked adhesive composition according to any of claims 1, 2, or 5 to 17.

34. A kit for manufacture of an adhesive, the kit comprising separate components, wherein admixture of the separate components forms an adhesive composition according to any of claims 1, 2 or 5 to 17.

35. A kit according to claim 34 that comprises separate first and second components, the first component comprising an extensin protein, the second component comprising:
a non-enzymatic bifunctional crosslinking agent; and/or
a phenol oxidase and a phenol hydroxylase and optionally a cofator;
wherein admixture of the first and second components forms a composition according to any of claims 1, 2 or 5 to 16.

## Patentansprüche

1. Zusammensetzung zur Verwendung als Klebstoff, umfassend:
ein Extensinprotein; und
ein nichtenzymatisches bifunktionelles Vernetzungsmittel; und/oder
eine Phenoloxidase und eine Phenolhydroxylase.

2. Zusammensetzung nach Anspruch 1, ferner umfassend einen Cofaktor, wenn die Zusammensetzung eine Phenoloxidase und eine Phenolhydroxylase umfasst.

3. Verfahren zur Bildung eines Klebstoffs, umfassend das Vermischen eines Extensinproteins mit einer Menge von:
einem nichtenzymatischen bifunktionellen Vernetzungsmittel und/oder einer Phenoloxidase und einer Phenolhydroxylase, die wirksam eine Vernetzung des Proteins bewirkt.

4. Verfahren nach Anspruch 3, wobei das Extensinprotein mit einem Cofaktor vermischt wird, wenn der Klebstoff eine Phenoloxidase und eine Phenolhydroxylase umfasst, wobei die Menge des Cofaktors, der Phenoloxidase und der Phenolhydroxylase wirksam die Vernetzung des Proteins bewirkt oder die Menge des Cofaktors, nichtenzymatischen bifunktionellen Vernetzungsmittels, der Phenoloxidase und Phenolhydroxylase wirksam die Vernetzung des Proteins bewirkt.

5. Zusammensetzung oder Verfahren nach Anspruch 2 oder 4, wobei der Cofaktor einen Phenolanteil umfasst, der eine Monohydroxyphenolgruppe und/oder eine Dihydroxyphenolgruppe umfasst.

6. Zusammensetzung oder Verfahren nach Anspruch 2, 4 oder 5, wobei der Cofaktor in Wasser löslich ist.

7. Zusammensetzung oder Verfahren nach Anspruch 5 oder 6, wobei der Cofaktor Catechin umfasst.

8. Zusammensetzung oder Verfahren nach einem der Ansprüche 5 bis 7, wobei der Cofaktor Catechol umfasst.

9. Zusammensetzung oder Verfahren nach einem der vorherigen Ansprüche, wobei das nichtenzymatische bifunktionelle Vernetzungsmittel Glutaraldehyd umfasst.

10. Zusammensetzung oder Verfahren nach einem der vorherigen Ansprüche, wobei das nichtenzymatische bifunktionelle Vernetzungsmittel ein Diisocyanat umfasst.

11. Zusammensetzung oder Verfahren nach Anspruch 10, wobei das Diisocyanat Trixene ist.

12. Zusammensetzung oder Verfahren nach einem der vorherigen Ansprüche, wobei das nichtenzymatische bifunktionelle Vernetzungsmittel ein Chinon umfasst.

13. Zusammensetzung oder Verfahren nach Anspruch 12, wobei das Chinon ein Benzochinon ist.

14. Zusammensetzung oder Verfahren nach einem der vorherigen Ansprüche, wobei die Phenoloxidase und die Phenolhydroxylase eine Tyrosinase ist.

15. Zusammensetzung oder Verfahren nach Anspruch 14, wobei die Tyrosinase eine Pilz-Tyrosinase ist.

16. Zusammensetzung oder Verfahren nach Anspruch 15, wobei die Pilz-Tyrosinase *Agaricus bisporus* Tyrosinase ist.

17. Zusammensetzung zur Verwendung als Klebstoff, umfassend:
ein Extensinprotein;
einen Cofaktor, umfassend eine Dihydroxyphenolgruppe;
eine Phenoloxidase; und bei Bedarf
ein nichtenzymatisches bifunktionelles Vernetzungsmittel.

18. Verfahren zur Bildung eines Klebstoffs, umfassend das Vermischen eines Extensinproteins mit einer Menge eines Cofaktors, umfassend eine Dihydroxyphenolgruppe, eine Phenoloxidase und bei Bedarf ein nichtenzymatisches bifunktionelles Vernetzungsmittel, das effektiv die Vernetzung des Proteins bewirkt.

19. Verwendung einer Zusammensetzung nach einem der Ansprüche 1, 2 oder 5-17 zum Verbinden von Substraten miteinander.

20. Verwendung nach Anspruch 19, wobei die Substrate nicht wasserabsorbierend sind.

21. Verwendung nach Anspruch 19, wobei die Substrate wasserabsorbierend sind.

22. Verwendung nach Anspruch 19, wobei die Substrate ein nicht wasserabsorbierendes Substrat und ein wasserabsorbierendes Substrat umfassen.

23. Verwendung nach Anspruch 20 oder 22, wobei das/die nicht wasserabsorbierende(n) Substrat(e) Metall und/oder Kunststoff umfassen.

24. Verwendung nach Anspruch 21 oder 22, wobei das/die wasserabsorbierende(n) Substrat(e) Holz und/oder Leder und/oder Baumwolle und/oder Papier und/oder Teppich und/oder Gewebe umfassen.

25. Verwendung nach Anspruch 19 als ein Bindemittel von Partikeln.

26. Verwendung nach Anspruch 25, wobei die Partikel Sand und/oder Glasfaser umfassen.

27. Verwendung nach Anspruch 19 als Unterschicht für eine Beschichtung.

28. Verwendung nach Anspruch 27, wobei die Beschichtung eine Anstrichfarbe ist.

29. Verwendung nach Anspruch 27, wobei die Beschichtung ein Klebstoff ist.

30. Verwendung nach Anspruch 19 als eine Naht zum Schließen einer Wunde.

31. Verwendung nach Anspruch 30 in einem Verfahren zum Schließen einer Wunde.

32. Verwendung nach Anspruch 19 als ein Geliermittel in Lebensmittelprodukten.

33. Pharmazeutische Zusammensetzung, umfassend einen pharmazeutisch aktiven Bestandteil und eine vernetzte Klebstoffzusammensetzung nach einem der Ansprüche 1, 2 oder 5 bis 17.

34. Kit zur Herstellung eines Klebstoffs, wobei der Kit separate Komponenten umfasst, wobei durch das Vermischen der separaten Komponenten eine Klebstoffzusammensetzung nach einem der Ansprüche 1, 2 oder 5 bis 17 gebildet wird.

35. Kit nach Anspruch 34, der separate erste und zweite Komponenten umfasst, wobei die erste Komponente ein Extensinprotein umfasst und die zweite Komponente folgendes umfasst: ein nichtenzymatisches bifunktionelles Vernetzungsmittel; und/oder eine Phenoloxidase und eine Phenolhydroxylase und bei Bedarf einen Cofaktor;
wobei durch das Vermischen der ersten und der zweiten Komponente eine Zusammensetzung nach einem der Ansprüche 1, 2 oder 5 bis 16 gebildet wird.

## Revendications

1. Composition à utiliser en qualité d'adhésif comprenant :
une protéine extensine; et
un agent de réticulation bifonctionnel non enzymatique; et/ou
une phénol-oxydase et une phénol-hydroxylase.

2. Composition selon la revendication 1, qui comprend en outre un cofacteur lorsque la composition comprend une phénol-oxydase et une phénol-hydroxylase.

3. Procédé pour former un adhésif, qui comprend ajouter/mélanger une protéine extensine avec une quantité soit : d'un agent de réticulation bifonctionnel non enzymatique; et/soit d'une phénol-oxydase et d'une phénol-hydroxylase efficace pour induire la réticulation de la protéine.

4. Procédé selon la revendication 3, dans lequel la protéine extensine est ajoutée/mélangée avec un cofacteur lorsque l'adhésif comprend une phénol-oxydase et une phénol-hydroxylase, la quantité du cofacteur, phénol-oxydase et phénol-hydroxylase étant efficace pour induire la réticulation de la protéine ou bien la quantité du cofacteur, agent de réticulation bifonctionnel non enzymatique, phénol-oxydase et phénol-hydroxylase étant efficace pour induire la réticulation de la protéine.

5. Composition ou procédé selon la revendication 2 ou 4, dans lesquels le cofacteur comprend une moitié phénolique qui comprend au moins l'un d'un groupe phénol monohydroxy ou d'un groupe phénol dihydroxy.

6. Composition ou procédé selon la revendication 2, 4 ou 5, dans lesquels le cofacteur est soluble dans l'eau.

7. Composition ou procédé selon la revendication 5 ou 6, dans lesquels le cofacteur comprend de la catéchine.

8. Composition ou procédé selon l'une quelconque des revendications 5 à 7, dans lesquels le cofacteur comprend du catéchol.

9. Composition ou procédé selon l'une quelconque des revendications précédentes, dans lesquels l'agent de réticulation bifonctionnel non enzymatique comprend du glutaraldéhyde.

10. Composition ou procédé selon l'une quelconque des revendications précédentes, dans lesquels l'agent de réticulation bifonctionnel non enzymatique comprend un diisocyanate.

11. Composition ou procédé selon la revendication 10, dans lesquels le diisocyanate est le Trixene.

12. Composition ou procédé selon l'une quelconque des revendications précédentes, dans lesquels l'agent de réticulation bifonctionnel non enzymatique comprend une quinone.

13. Composition ou procédé selon la revendication 12, dans lesquels la quinone est une benzoquinone.

14. Composition ou procédé selon l'une quelconque des revendications précédentes, dans lesquels la phénol-oxydase et la phénol-hydroxylase est une tyrosinase.

15. Composition ou procédé selon la revendication 14, dans lesquels la tyrosinase est une tyrosinase de champignon.

16. Composition ou procédé selon la revendication 15, dans lesquels la tyrosinase de champignon est la tyrosinase *Agaricus bisporus*.

17. Composition à utiliser en qualité d'adhésif, qui comprend :
une protéine extensine;
un cofacteur comprenant un groupe phénol dihydroxy;
une phénol-oxydase; et facultativement
un agent de réticulation bifonctionnel non enzymatique.

18. Procédé pour former un adhésif, qui comprend ajouter/mélanger une protéine extensine avec une quantité d'un cofacteur comprenant un groupe phénol dihydroxy, une phénol-oxydase et facultativement un agent de réticulation bifonctionnel non enzymatique efficace pour induire la réticulation de la protéine.

19. Utilisation d'une composition selon l'une quelconque des revendications 1, 2, ou 5-17, pour lier des substrats ensemble.

20. Utilisation selon la revendication 19, dans laquelle les substrats sont hydrophobes.

21. Utilisation selon la revendication 19, dans laquelle les substrats sont hydrophiles.

22. Utilisation selon la revendication 19, dans laquelle les substrats comprennent un substrat hydrophobe et un substrat hydrophile.

23. Utilisation selon la revendication 20 ou 22, dans laquelle le substrat ou les substrats hydrophobes comprennent au moins l'un parmi le métal ou le plastique.

24. Utilisation selon la revendication 21 ou 22, dans laquelle le substrat ou les substrats hydrophiles comprennent au moins l'un parmi le bois, le cuir, le coton, le papier, le tapis ou le textile.

25. Utilisation selon la revendication 19 en qualité de liant pour des matières particulaires.

26. Utilisation selon la revendication 25, dans laquelle les matières particulaires comprennent au moins l'un parmi le sable ou la fibre de verre.

27. Utilisation selon la revendication 19 en qualité de sous-couche d'un revêtement.

28. Utilisation selon la revendication 27, dans laquelle le revêtement est une peinture.

29. Utilisation selon la revendication 27, dans laquelle le revêtement est un adhésif.

30. Utilisation selon la revendication 19 en qualité de suture pour fermer une plaie.

31. Utilisation selon la revendication 30 dans un procédé pour fermer une plaie.

32. Utilisation selon la revendication 19 en qualité d'agent gélifiant dans des produits alimentaires.

33. Composition pharmaceutique comprenant un ingrédient pharmaceutiquement actif et une composition adhésive réticulée selon l'une quelconque des revendications 1, 2 ou 5 à 17.

34. Kit pour la fabrication d'un adhésif, le kit comprenant des composants séparés, dans lequel une addition/mélange des composants séparés forme une composition adhésive selon l'une quelconque des revendications 1, 2 ou 5 à 17.

35. Kit selon la revendication 34, qui comprend des premier et deuxième composants séparés, le premier composant comprenant une protéine extensine, le deuxième composant comprenant : un agent de réticulation bifonctionnel non enzymatique; et/ou une phénol-oxydase et une phénol-hydroxylase et facultativement un cofacteur;
dans lequel l'addition/mélange des premier et deuxième composants forme une composition selon l'une quelconque des revendications 1, 2 ou 5 à 16.
